# EUROPÄISCHE PATENTSCHRIFT

(11) **EP 3 688 867 B1**
(45) Veröffentlichungstag und Bekanntmachung des Hinweises auf die Patenterteilung: **10.01.2024**
(21) Anmeldenummer: 18782903.1
(22) Anmeldetag: 26.09.2018
(51) Int. Cl.: H02K 49/10, A61M 1/14, F04D 15/00, H02J 7/00, H02K 7/14, H02K 11/20, H02K 11/33

(54) **MEDIZINISCHER PUMPENANTRIEB, PUMPE UND MEDIZINISCHE BEHANDLUNGSVORRICHTUNG**
MEDICAL PUMP DRIVE, PUMP ANDTREATMENT DEVICE
MÉCANISME D'ENTRAÎNEMENT DE POMPE MÉDICALE, POMPE ET DISPOSITIF DE TRAITEMENT

(30) Priorität: 29.09.2017 DE 102017122804
(43) Veröffentlichungstag der Anmeldung: 05.08.2020
(73) Patentinhaber: Fresenius Medical Care Deutschland GmbH, 61352 Bad Homburg (DE)
(72) Erfinder: HEIDE, Alexander, 65817 Eppstein (DE); PETERS, Arne, 61352 Bad Homburg (DE); NIKOLIC, Dejan, 65812 Bad Soden (DE); KLEWINGHAUS, Juergen, 61440 Oberursel (DE)
(74) Vertreter: Bobbert & Partner Patentanwälte PartmbB
(86) Internationale Anmeldenummer: PCT/EP2018/076074
(87) Internationale Veröffentlichungsnummer: WO 2019/063593

(56) Entgegenhaltungen:
- EP-A2- 1 293 224
- WO-A1-2015/098709
- US-A- 4 816 151
- US-A1- 2002 161 274
- US-A1- 2005 075 696
- US-A1- 2013 301 845
- US-A1- 2015 367 049

## Beschreibung

Die vorliegende Erfindung betrifft einen medizinischen Pumpenantrieb gemäß Anspruch 1, eine Pumpe gemäß Anspruch 11 sowie ein Set hieraus gemäß Anspruch 15. Sie umfasst ferner einen Schlauchsatz gemäß Anspruch 16 und eine medizinische Behandlungsvorrichtung (kurz auch: Behandlungsvorrichtung) gemäß Anspruch 17. Außerdem werden ein Verfahren zum Laden eines Akkus und ein Verfahren zum Zusammenbringen zweier Abschnitte der Pumpe offenbart.

Aus der Praxis ist die extrakorporale Blutbehandlung bekannt. Dabei wird dem Patienten Blut entnommen, das entlang eines Blutkreislaufs oder Schlauchsatzes extrakorporal und - im Beispiel einer Dialysebehandlung - z. B. durch einen Blutfilter geführt wird. Der Blutfilter weist eine Blutkammer, durch welche Blut geführt wird, und eine Dialysierflüssigkeitskammer, durch welche Dialysierflüssigkeit geführt wird, auf. Beide Kammern sind durch eine semi-permeable Membran voneinander getrennt. Blut und Dialysierflüssigkeit werden zumeist im Gegenstromprinzip durch den Blutfilter geleitet. Das Blut wird im Blutfilter gereinigt, die Dialysierflüssigkeit gilt bei ihrem Austritt aus dem Blutfilter als verbraucht und wird, nun als Dialysat bezeichnet, verworfen. Neben dem Dialysat umfasst das zu verwerfende Fluid auch Filtrat, welches Wasser, das dem Blut im Blutfilter entzogen wurde, umfasst. Filtrat und Dialysat werden im Folgenden einzeln oder gemeinsam vereinfacht als Effluent bezeichnet.

Das Effluent wird in der Praxis mittels einer Effluentzulaufleitung einem Effluentbeutel zugeführt und darin zunächst aufbewahrt. Nach Beendigung der Blutbehandlung, oder in Beutelleerintervallen (Intervalle, in welchen der Beutel geleert wird) während der Blutbehandlung, wird das Effluent aus dem Effluentbeutel, der über ein Waschbecken oder einen Ausguss gehalten wird, in diesen hinein verworfen.

Aus der WO 2015/098709 A1 ist Zentrifugalpumpensystem bekannt.

In der US 2002/0161274 A1 sind Verfahren, Systeme und Vorrichtungen im Zusammenhang mit implantierbaren Pumpen offenbart.

Eine implantierbare Blutpumpe mit integrierter Steuervorrichtung ist aus der US 2015/0367049 A1 bekannt.

Ein Rotorantrieb für medizinische Einwegartikel ist in der US 4 816 151 A offenbart.

Aus der US 2005/075696 A1 sind eine induktiv ladbare externe Energiequelle, ein Ladegerät, ein System und ein Verfahren für ein transkutanes Induktionsladegerät für eine implantierbare medizinische Vorrichtung bekannt.

In der US 2013/0301845 A1 sind Ohrstöpsel, Verfahren und Sets zum Erzielen und Aufrechterhalten von Schlafqualität offenbart.

Ein Spender für Medien, mittels welchem das tatsächliche Volumen an ausgetragenem Medium möglichst genau bestimmbar ist, ist aus der EP 1 293 224 A2 bekannt.

Eine Aufgabe der vorliegenden Erfindung ist es, einen Pumpenantrieb zum Entleeren eines Effluentbeutels zum Einsatz bei der Blutbehandlung anzugeben.

Ferner sollen eine Pumpe, ein Set, ein Schlauchsatz und eine medizinische Behandlungsvorrichtung, die insbesondere eine Blutbehandlungsvorrichtung sein kann, angegeben werden.

Weiter sollen ein Verfahren zum Laden des Akkus des Pumpenantriebs sowie ein Verfahren zum Verbinden eines erfindungsgemäßen Pumpenantriebs mit einem Pumpenabschnitt, der einen Pumpenrotor enthält, offenbart werden.

Die erfindungsgemäße Aufgabe wird durch einen medizinischen Pumpenantrieb mit den Merkmalen des Anspruchs 1 gelöst. Zudem wird sie gelöst durch eine Pumpe gemäß Anspruch 11,sowie ein Set hieraus gemäß Anspruch 15. Zur Lösung der erfindungsgemäßen Aufgabe gehört ferner ein Schlauchsatz gemäß Anspruch 16 und eine Behandlungsvorrichtung gemäß Anspruch 17. Außerdem werden ein Verfahren zum Laden eines Akkus und ein Verfahren zum Zusammenbringen zweier Abschnitte einer Pumpe offenbart.

Alle mit den offenbarten Verfahren erzielbaren Vorteile lassen sich in bestimmten erfindungsgemäßen Ausführungsformen ungeschmälert auch mit den erfindungsgemäßen Vorrichtungen erzielen.

Die vorliegende Erfindung betrifft einen medizinischen Pumpenantrieb. Dieser umfasst wenigstens ein Gehäuse und einen, im Gehäuse vorgesehenen, Pumpenmotor. Der Pumpenantrieb umfasst ferner wenigstens eine wiederladbare Spannungsquelle zum Speichern von elektrischer Energie, oder einen Akku. Diese dienen der Spannungsversorgung des Pumpenmotors.

Außerdem ist der Pumpenantrieb mit wenigstens einem Magnetabschnitt ausgestaltet, z. B. ausgestaltet als Spulen mit Eisenkern. Dieser dient zum magnetischen Ankoppeln und/oder magnetischen Antreiben eines magnetisch antreibbaren Pumpenrotors. Der Antrieb ist vorzugweise kontaktlos.

Schließlich weist der Pumpenantrieb wenigstens eine Steuerelektronik zu seinem Steuern oder Regeln auf.

Alle Komponenten des Pumpenantriebs, insbesondere die vorstehend genannten, können optional gemeinsam im oder vom Gehäuse aufgenommen sein. Sie können sich in einigen Ausführungsformen somit das Gehäuse teilen. In manchen Ausführungsformen weist der Pumpenantrieb genau ein Gehäuse auf, das seine Komponenten von einem Äußeren des Pumpenantriebs trennt.

Die vorliegende Erfindung betrifft weiter eine Pumpe. Sie weist einen erfindungsgemäßen Pumpenantrieb und wenigstens einen Pumpenabschnitt auf, welcher seinerseits einen Pumpenrotor aufweist. Der Pumpenabschnitt mit dem Pumpenrotor ist vorzugsweise Teil eines Schlauchsatzes und/oder eines Disposables.

Die vorliegende Beschreibung offenbart ferner eine Induktionsladestation zum Laden des Akkus des Pumpenantriebs. Die Induktionsladestation besitzt optional eine Steuerelektronik, welche konfiguriert ist, um das Aufliegen des Pumpenantriebs auf der Induktionsladestation zu erkennen und den Akku zu laden.

Die vorliegende Erfindung betrifft ein Set. Das Set umfasst wenigstens einen erfindungsgemäßen Pumpenantrieb und/oder eine erfindungsgemäße Pumpe. Ferner weist das Set eine Induktionsladestation, welche konfiguriert ist, um das Aufliegen des erfindungsgemäßen Pumpenantriebs auf der Induktionsladestation zu erkennen und den Akku des erfindungsgemäßen Pumpenantriebs zu laden, und/oder ein externes Gerät auf. Induktionsladestation und/oder externes Gerät sind so konfiguriert, dass sie über ein Wireless-Modul mit der Steuerelektronik des Pumpenantriebs in Signalverbindung stehen, um Signale, Zustände, Funktionen und/oder Einstellungen der Steuerelektronik und/oder des Akkus empfangen und anzeigen zu können.

Die Induktionsladestation kann eine Plattform oder Aufstellebene für den Pumpenantrieb sein oder aufweisen.

Die vorliegende Erfindung betrifft einen Schlauchsatz, insbesondere einen medizinischen Schlauchsatz, welcher wenigstens einen Pumpenabschnitt mit einem Pumpenrotor aufweist. Der Pumpenabschnitt ist vorgesehen, um funktionell mit einem Pumpenantrieb, insbesondere einem erfindungsgemäßen Pumpenantrieb, verbunden oder zusammengebracht zu werden. Der Pumpenabschnitt, und optional auch alle anderen Abschnitte des Schlauchsatzes, weisen keine Einrichtung(en) zum Verbinden des Pumpenabschnitts mit dem Pumpenantrieb auf, abgesehen von einer optionalen Magnetverbindung und/oder Nutzung der Schwerkraft.

Die vorliegende Erfindung betrifft eine Behandlungsvorrichtung, welche eine erfindungsgemäße Pumpe, einen erfindungsgemäßen Schlauchsatz und/oder ein erfindungsgemäßes Set aufweist oder hiermit jeweils verbunden ist. Die Behandlungsvorrichtung kann eine Blutbehandlungsvorrichtung sein. Sie kann alternativ an anderer Stelle der Patientenversorgung als der Blutbehandlung zum Einsatz kommen. Sie kann der Analyse, Therapie, Diagnostik oder Behandlung dienen.

Ist die Behandlungsvorrichtung als Blutbehandlungsvorrichtung ausgestaltet, so kann sie eine Blutpumpe aufweisen. Die Blutpumpe fördert während der Behandlung des Patienten Blut durch Abschnitte des extrakorporalen Blutkreislaufs und in Richtung zum Blutfilter oder Dialysator. Von diesem wegführend wird das Blut durch Abschnitte des extrakorporalen Blutkreislaufs hindurch dem Patienten zurückgegeben. Eine venöse Blutkammer und ein Blasenfänger können ebenso wie Drucksensoren Teil des extrakorporalen Blutkreislaufs und/oder der Blutbehandlungsvorrichtung sein.

Die vorliegende Beschreibung offenbart ein Verfahren zum Laden des Akkus des Pumpenantriebs. Dieses Verfahren umfasst das Drehen oder Ausrichten des Pumpenantriebs (z. B. in der Hand, in der Luft, usw.) derart, dass jenes Ende, welchem die Induktionsladespule zugeordnet ist, nach unten zeigt. Das Verfahren umfasst weiter das Aufstellen oder Aufsetzen des so ausgerichteten Pumpenantriebs in einer Bewegungsrichtung, welche zumindest auch von oben nach unten führt, auf eine Aufstellfläche einer induktiven Ladestation, insbesondere der erfindungsgemäßen Induktionsladestation.

Die vorliegende Beschreibung offenbart ein Verfahren zum Zusammenbringen eines Pumpenantriebs mit einem Pumpenabschnitt, welcher einen Pumpenrotor aufweist, mit einem optionalen Drehen oder Ausrichten des Pumpenantriebs (z. B. in der Hand, in der Luft, usw.) derart, dass jenes Ende, welches den Magnetabschnitt aufweist oder diesem zugeordnet ist, dabei nach unten zeigt. Das Verfahren umfasst ferner ein Aufstellen oder Aufsetzen des so ausgerichteten Pumpenantriebs in einer Bewegungsrichtung, welche zumindest auch von oben nach unten führt, auf den Pumpenabschnitt, welcher einen Pumpenrotor aufweist. Dieses Verfahren ergibt eine vollständige und/oder funktionsfähige Pumpe.

Unter einem Zusammenbringen kann ein Zusammenfügen des Pumpenantriebs und des Pumpenabschnitts oder Aufsetzen des Pumpenantriebs auf den Pumpenabschnitt derart verstanden werden, dass sie zusammen die funktionsfertige Pumpe ergeben.

Erfindungsgemäße Ausführungsformen können manche, einige oder alle der folgenden Merkmale in beliebiger Kombination aufweisen, soweit dies für den Fachmann nicht erkennbar technisch unmöglich ist. Vorteilhafte Weiterbildungen der vorliegenden Erfindung sind jeweils auch Gegenstand der Unteransprüche.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mitumfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Wenn hierin von einer Ausführungsform die Rede ist, so stellt dies eine erfindungsgemäße, beispielhafte Ausführungsform dar.

Wenn hierin von "programmiert" oder "konfiguriert" die Rede ist, so ist auch offenbart, diese Begriffe gegeneinander auszutauschen.

Wann immer hierin von "oben" oder von "unten" die Rede ist, versteht der Fachmann hierunter die Anordnung in den hier angehängten Figuren und/oder im Gebrauchszustand. "Unten" ist dem Erdmittelpunkt oder dem unteren Rand der Figur näher als "oben".

Wann immer hierin eine Eignung oder ein Verfahrensschritt genannt ist umfasst die vorliegende Erfindung auch eine entsprechende Programmierung oder Konfigurierung einer geeigneten Vorrichtung oder eines Abschnitts hiervon.

In einigen Ausführungsformen besteht die Pumpe aus Pumpenantrieb und Pumpenabschnitt, welcher wiederum mit Schläuchen verbunden sein kann. Diese Schläuche können zur Pumpe zählen. In manchen Ausführungsformen zählen sie nicht zur Pumpe.

In manchen Ausführungsformen weist der Pumpenantrieb eine Induktionsladespule zum Laden der Spannungsquelle oder des Akkus auf.

In einigen Ausführungsformen weist das Gehäuse einen Aufstellabschnitt zum Aufstellen des Pumpenantriebs auf einer Aufstellfläche auf.

In manchen Ausführungsformen weist das Gehäuse einen Verbindungsabschnitt zum, insbesondere funktionellen, Verbinden des Pumpenantriebs mit einem Pumpenabschnitt, welcher einen Pumpenrotor beinhaltet, auf. Der Verbindungsabschnitt kann hierbei eine Kavität, eine oder mehrere Öffnungen, eine oder mehrere Sacköffnungen oder dergleichen sein. Letztere können, vorzugsweise exklusiv, der Aufnahme einer Erhebung des Pumpenabschnitts dienen, z. B. durch reines Einstecken oder Einführen der Erhebung in den Verbindungsabschnitt. Der Verbindungsabschnitt kann daher eine Einstecköffnung sein.

In einigen Ausführungsformen liegen der Aufstellabschnitt und der Verbindungsabschnitt an gegenüberliegenden Enden des Gehäuses oder sind diesen zugeordnet.

In manchen Ausführungsformen weist der Pumpenantrieb wenigstens eine, insbesondere farbige, Leuchteinrichtung auf.

Diese kann insbesondere als Ring, insbesondere als LED oder LED-Farbring, ausgestaltet sein.

Die Leuchteinrichtung kann insbesondere im oder am Gehäuse positioniert sein.

In einigen Ausführungsformen ist die Steuerelektronik des Pumpenantriebs mit einem Wireless-Modul ausgestaltet oder mit einem solchen verbunden.

In einigen Ausführungsformen ist die Steuerelektronik ausgestaltet, programmiert und/oder konfiguriert zum Abgeben von Signalen, die auf einem oder mehreren externen Geräten empfangen werden oder werden können, ggf. auch gleichzeitig, und die Funktionen und/oder Einstellungen der Steuerelektronik bzw. die Zustände oder Funktionen der Pumpe oder des Akkus anzeigen oder hierüber informieren.

In einigen Ausführungsformen ist das externe Gerät ein Smartphone oder ein anderes Handgerät oder mobiles Gerät.

Ein Wireless-Modul kann eine Computer-Hardware sein, mit der sich ein Rechner oder eine Steuerelektronik mittels einer Schnittstelle (z. B. PCI, USB, PCMCIA, PC Card) mit einem Funknetz (WLAN) verbinden lässt.

Erfindungsgemäß ist die Steuerelektronik mit wenigstens einem Bewegungssensor ausgestaltet oder verbunden. Dieser kann integriert oder verbunden sein, damit die entsprechend programmierte Steuerelektronik mittels diesem die Position oder Bewegungsrichtung ermitteln kann.

In manchen Ausführungsformen ist die Steuerelektronik konfiguriert, den laufenden Ladevorgang mit einer oder mehreren Farben, z. B. als Änderung der Farben von Rot über Gelb nach Grün, zu signalisieren bzw. den aktuellen Akkuladezustand anzuzeigen.

In einigen Ausführungsformen ist die Steuerelektronik konfiguriert, den Akkuladezustand qualitativ oder quantitativ anzuzeigen, etwa als "voll", "leer", per Prozentangabe wie z. B. "90%", "einsatzbereit", "nicht einsatzbereit", usw. Diese Angabe kann z. B. per Display und/oder per Übertragung per Wireless-Modul auf einem externen Gerät erfolgen.

In manchen Ausführungsformen ist die Steuerelektronik konfiguriert, die Leuchteinrichtung bei oder nach Erreichen des Zustandes der vollständigen Ladung oder bei oder nach Erreichen eines vorbestimmten Ladezustands der Spannungsquelle oder des Akkus auszuschalten.

In manchen Ausführungsformen ist die Steuerelektronik konfiguriert, auf das Erkennen einer Bewegung des Pumpenantriebs mittels des Bewegungssensors hin von einem ersten Zustand der Steuerelektronik oder des Pumpenantriebs in einen zweiten Zustand überzugehen.

Der erste Zustand kann ein sleep-Modus, ein inaktiver Modus und/oder ein Zustand eines niedrigeren Stromverbrauchs (verglichen mit dem Stromverbrauch oder mittleren Stromverbrauch im zweiten Zustand) sein. Im ersten Zustand kann beispielsweise der Lagerstrom zum Antreiben eines Magnetlagers, bspw. für den Magnetabschnitt des Pumpenantriebs, Null sein.

Der zweite Zustand kann ein aktiver Modus, ein awake-Modus und/oder ein Zustand eines höheren Stromverbrauchs (verglichen mit dem Stromverbrauch oder mittleren Stromverbrauch im ersten Zustand) sein. Im zweiten Zustand kann beispielsweise das Magnetlager, bspw. der Magnetabschnitt des Pumpenantriebs, mit Spannung beaufschlagt sein und Strom verbrauchen.

Das Erkennen einer Bewegung des Pumpenantriebs mittels des Bewegungssensors kann auf ein Erkennen der Bewegung des Pumpenantriebs im Raum gerichtet sein.

So kann der Bewegungssensor oder die nachfolgende Steuerelektronik ausgestaltet und/oder konfiguriert sein, anzuzeigen, dass der Pumpenantrieb bewegt wurde, etwa, weil er von der Induktionsladestation oder einem anderen Ort aufgenommen und in der Hand gehalten wird.

Zugleich kann mittels des Bewegungssensors optional festgestellt werden, dass der Pumpenantrieb von der Induktionsladestation abgenommen, gedreht und umgekehrt in einer von oben nach unten gerichteten Bewegung auf den Pumpenabschnitt mit den Pumpenrotor aufgesetzt wurde. Dies kann als Hinweis auf die anstehende Verwendung des Pumpenantriebs als Teil der Pumpe verstanden werden und optional automatisch einen Übergang vom ersten Zustand in den zweiten Zustand veranlassen oder bewirken.

Eine von oben nach unten gerichtete Bewegung des Pumpenantriebs kann - wie optional andere Bewegungen auch - als Bewegungsablauf hinterlegt sein. Die Pumpe kann konfiguriert sein, bei Erkennen dieses Bewegungsablaufs - z. B. bei Abschluss des hinterlegten Bewegungsablaufs - automatisch zu pumpen zu beginnen. Dieser Bewegungsablauf kann wie auch weitere in einer Referenzdatenbank hinterlegt sein.

Andere Funktionen, die mit konkreten Bewegungen des Pumpenantriebs zusammenhängen, können ebenfalls hinterlegt sein und mittels der Steuerelektronik bei Erkennen der konkreten Bewegung gestartet, angehalten, ausgeführt, usw. werden.

Auch vorbestimmte Kunstbewegungen ähnlich Gesten, die mit dem Pumpenantrieb in der Hand vom Benutzer ausgeführt werden und vom Bewegungssensor registriert werden können, sind in ihrer Implementierung von der Erfindung umfasst. Die Steuerelektronik kann zum Erkennen jeder der hierin genannten Bewegungen, und weiterer, entsprechend konfiguriert sein.

In einigen Ausführungsformen wird im zweiten Zustand der Ladezustand mittels der Leuchteinrichtung angezeigt.

In einigen Ausführungsformen wird im zweiten Zustand angezeigt, dass eine Bewegung erkannt wurde.

In einigen Ausführungsformen erfolgt mittels der Leuchteinrichtung im zweiten Zustand eine andere Leuchtemission als im ersten Zustand.

In manchen Ausführungsformen besteht die Pumpe aus einem Pumpenantrieb, wobei die Steuerelektronik konfiguriert ist, insbesondere auf das Erkennen einer Bewegung des Pumpenantriebs mittels des Bewegungssensors hin, den Magnetabschnitt des Pumpantriebs zu aktivieren und/oder mit Lagerspannung oder -strom zu vorsorgen bzw. optional in den zweiten Zustand überzugehen und den Pumpenantrieb und/oder den Magnetabschnitt mit Spannung oder Strom zu versorgen.

In manchen Ausführungsformen ist die Steuerelektronik des Pumpenantriebs konfiguriert, den Ladezustand mittels der Leuchteinrichtung anzuzeigen.

In einigen Ausführungsformen ist die Steuerelektronik konfiguriert, den Ladezustand erst dann anzuzeigen, wenn sie mittels des Bewegungssensors eine Bewegung des Pumpenantrieb erkannt hat.

In einigen Ausführungsformen ist die Steuerelektronik konfiguriert, insbesondere auf das Erkennen eines Pumpenabschnitts mit dem Pumpenrotor oder auf das Erkennen eines Pumpenrotors, z. B. mittels eines Hallsensors, hin, den Magnetabschnitt des Pumpantriebs zu rotieren, z. B. im zweiten Zustand, insbesondere auf eine vorbestimmte Drehzahl, insbesondere unter Anzeigen der stattfindenden Rotation mittels der Leuchteinrichtung, z. B. in blau, z. B. mittels umlaufender Lichtemission.

In einigen Ausführungsformen ist die Steuerelektronik konfiguriert, mittels eines, insbesondere Low-power, Bewegungssensors eine Bewegung des Pumpenantriebs zu erkennen und in einen zweiten oder aktiven Zustand überzugehen.

In manchen Ausführungsformen ist die Steuerelektronik des Pumpenantriebs konfiguriert, insbesondere auf das Erkennen eines versiegenden, abnehmenden oder versiegten Flüssigkeitsstroms hin, welcher durch die erfindungsgemäße Pumpe fließt, die maximale oder die voreingestellte Drehzahl zu reduzieren. Das Reduzieren erfolgt vorzugsweise auf eine Drehzahl, welche ein Gleichgewicht herstellt zwischen der mittels der Pumpe gepumpten Flüssigkeit einerseits und der Flüssigkeit, welche in die Pumpe mittels Schwerkraft oder anderen Gründen nachläuft, andererseits.

In einigen Ausführungsformen ist die Steuerelektronik konfiguriert, versiegende, abnehmende oder versiegte Flüssigkeitsströme anhand eines Sensors und/oder anhand von Änderungen der Antriebs- oder Lagerströme zu erkennen. Als Lagerströme werden hierin jene Ströme der Stromversorgung bezeichnet, welche von Magnetlagern oder vom Magnetantrieb zum Erzeugen der Lagerkraft und Rotation durch geregelte Elektromagneten benötigt oder abgerufen werden.

Ein hiermit verbundener Vorteil kann darin bestehen, dass es keines Rückschlagventils bedarf, um zu verhindern, dass Flüssigkeit durch die Pumpe hindurch entgegen einer bestimmungsgemäßen Pumprichtung strömt, also in einer Richtung vom Auslass zum Einlass der Pumpe.

In einigen Ausführungsformen ist die Steuerelektronik des Pumpenantriebs konfiguriert, einen Hinweis auf eine erfolgte Bewegung des Pumpenantriebs an ein externes Gerät zu übermitteln.

In manchen Ausführungsformen beträgt die bevorzugte elektrischen Nennleistung zwischen 25 und 40 Watt (W), insbesondere 30 bis 35 W, ganz besonders 32 W.

In einigen Ausführungsformen beträgt die Betriebsspannung des elektrischen Antriebs der Pumpe 24 Volt (V).

In manchen Ausführungsformen weist der Pumpenabschnitt eine Einrichtung zum lösbaren Befestigen des Pumpenabschnitts an einem Gehäuseabschnitt oder an einem anderen Abschnitt einer Behandlungsvorrichtung, insbesondere Blutbehandlungsvorrichtung, insbesondere im Fußbereich dieser Vorrichtung, auf.

In einigen Ausführungsformen ist die Einrichtung zum lösbaren Befestigen des Pumpenabschnitts eine Klemmeinrichtung oder eine Rast- oder Clipeinrichtung oder weist eine solche auf.

In einigen Ausführungsformen hat die Einrichtung zum lösbaren Befestigen des Pumpenabschnitts, die optional eine Klemmeinrichtung oder eine Rast- oder Clipeinrichtung ist, einen gebogenen Verlauf.

Der gebogene Verlauf kann die Form eines entlang seiner Längserstreckung gekrümmten Halbkanals haben.

In manchen Ausführungsformen hat die Pumpe keine Einrichtung zum Verbinden des Pumpenantriebs mit dem Pumpenabschnitt, welcher einen Pumpenrotor trägt, abgesehen von einer optionalen Magnetverbindung und/oder der Nutzung der Schwerkraft, aufgrund welcher der Pumpenantrieb auf dem Pumpenabschnitt liegt.

In einigen Ausführungsformen weist der Pumpenantrieb, der Pumpenabschnitt und/oder ein anderer Bestandteil der Pumpe jedenfalls keine mechanische Verbindungseinrichtung zum Verbinden des Pumpenantriebs mit dem Pumpenabschnitt auf, außer der Aufstellfläche des Pumpenabschnitts, auf welche der Pumpenantrieb zum Erzielen der funktionsfähigen Pumpe aufgestellt wird.

Eine Verbindungseinrichtung zum Verbinden von Pumpenantrieb und Pumpenabschnitt könnte eine Klippverbindung, eine Rastverbindung, eine Schraubverbindung oder eine andere form- und/oder kraftschlüssige Verbindung sein. Eine Verbindungeinrichtung kann in manchen Ausführungsformen nicht allein eine Kavität oder eine Öffnung zum Einschieben einer Erhebung sein. Eine Verbindungseinrichtung ist in manchen Ausführungsformen nicht der hierin genannte Verbindungsabschnitt. In einigen Ausführungsformen ist der Verbindungsabschnitt frei von bewegbaren Elementen zum Halten des Pumpenantriebs am Pumpenabschnitt wie z. B. einer Rastzunge, einem elastischen Klick- oder Klippabschnitt, einem Schraubenabschnitt oder dergleichen und/oder hierzu korrespondierenden Strukturen wie einer Rastnase, einem Gewindeabschnitt, einem Klick- oder Klipp-Gegenabschnitt oder dergleichen zur Aufnahme oder zum Halten Ersterer.

Ein hiermit erzielbarer Vorteil kann darin bestehen, dass der Pumpenantrieb zu jeder Zeit (auch im laufenden Pumpvorgang) einfach nach oben abgezogen oder abgehoben werden kann. Dies kann insbesondere einhändig erfolgen, insbesondere wenn keine mechanischen Verbindungseinrichtungen zuvor geöffnet werden müssen, da letztere in diesen Ausführungsformen nicht vorgesehen sind.

In manchen Ausführungsformen ist der Pumpenabschnitt mit seinem Flüssigkeitseinlass oder Fluideinlass mit einem Schlauchabschnitt verbunden, der von einem Effluentbeutel zur Pumpe führt.

In einigen Ausführungsformen ist der Pumpenabschnitt mit seinem Flüssigkeitsauslass oder Fluidauslass mit einem Schlauchabschnitt verbunden, der zu einem Ablass, Ausgussbecken oder Ausguss zum Verwerfen des Effluents führt.

In manchen Ausführungsformen ist der Pumpenrotor magnetisch gelagert und/oder magnetisch angetrieben. Diese Art der Lagerung oder des Antriebs kann dem Schutz des Patienten vor einem Stromschlag dienen.

Der Magnetabschnitt des Pumpenrotors kann ein Permanentmagnet sein.

In manchen Ausführungsformen ist der Pumpenrotor ein Impellerpumpkopf.

In einigen Ausführungsformen ist der Pumpenantrieb kein fester Teil einer Behandlungsvorrichtung, insbesondere Blutbehandlungsvorrichtung. Er kann gleichwohl mit der Behandlungsvorrichtung lösbar verbunden werden oder sein, vorzugsweise ohne Kabelverbindung und/oder Signalleiterverbindung zur Behandlungsvorrichtung.

In manchen Ausführungsformen wird der Betrieb des Pumpenantriebs manuell begonnen und/oder beendet.

In gewissen Ausführungsformen ist die Blutbehandlungsvorrichtung als Hämodialysevorrichtung, Hämofiltrationsvorrichtung oder als Hämodiafiltrationsvorrichtung ausgestaltet, insbesondere als eine Vorrichtung für die chronische Nierenersatztherapie oder für die kontinuierliche Nierenersatztherapie (CRRT = continuous renal replacement therapy).

In einigen Ausführungsformen umfasst die erfindungsgemäße Behandlungsvorrichtung die Induktionsladestation für den Akku des Pumpenantriebs der Pumpe. Der Akku kann eine Niederspannungsquelle oder Schwachstromquelle sein.

In manchen Ausführungsformen beträgt die Speicherkapazität des Akkus des Pumpenantriebs zwischen 800 mAh und 1800 mAh, bevorzugt zwischen 1000 mAh und 1500 mAh, ganz besonders bevorzugt etwa oder genau 1100 mAh.

In manchen Ausführungsformen steht der Fluideinlass des Pumpenabschnitts mittels Schlauchs in Fluidverbindung mit einem Effluentbeutel.

In einigen Ausführungsformen ist eine Steuer- oder Regelvorrichtung der Behandlungsvorrichtung zum Umschalten der Umschaltvorrichtung derart, dass der Effluentbeutel mal mit der Effluentzulaufleitung und mal mit der Effluentablaufleitung verbunden wird, nicht konfiguriert oder programmiert.

In einigen Ausführungsformen ist die Steuerelektronik der Behandlungsvorrichtung konfiguriert, um auf ein Signal, welches das Umschalten einer Umschalteinrichtung, z. B. einer Mehrwegeventileinrichtung, welche in das Schlauchsystem mit der Effluentablaufleitung integriert ist, anzeigt, die Pumpe automatisch zu starten oder zu stoppen, je nach Signal.

Erfindungsgemäß wird ferner zusätzlich zum hierin offenbarten medizinischen Pumpenantrieb ein Pumpenantrieb vorgeschlagen, welcher jede beliebige Kombination von Merkmalen aufweist, die hierin für den erfindungsgemäßen medizinischen Pumpenantrieb offenbart sind. In diesen Ausführungsformen ist der erfindungsgemäße Pumpenantrieb jedoch kein medizinischer Pumpenantrieb, der z. B. zur Patientenversorgung, Zelltherapie oder dergleichen zum Einsatz kommt, sondern ist zu seinem Einsatz in einem Bereich außerhalb der Medizin oder der Förderung von medizinischen Fluiden vorgesehen. Solche Bereiche können das Bioprocessing, die Herstellung von Kosmetik, Papier oder Nahrung, Arzneimittelherstellung und anderes umfassen. Solche Bereiche können sich auch auf den Maschinenbau, insbesondere auf den Bereich von Haushalts- oder Küchengeräten erstrecken. Dies gilt auch für die unter Verwendung des Pumpenantriebs zusammengefügte Pumpe.

Erfindungsgemäß wird ferner zusätzlich zur hierin offenbarten medizinischen Behandlungsvorrichtung eine Vorrichtung vorgeschlagen, welche jede beliebige Kombination von Merkmalen aufweist, die hierin für die erfindungsgemäße medizinischen Behandlungsvorrichtung offenbart sind. In diesen Ausführungsformen ist die erfindungsgemäße Vorrichtung jedoch keine medizinische Behandlungsvorrichtung, die z. B. zur Patientenversorgung, Zelltherapie oder dergleichen zum Einsatz kommt, sondern ist zu ihrem Einsatz in einem Bereich außerhalb der Medizin oder der Förderung von medizinischen Fluiden vorgesehen. Solche Bereiche können das Bioprocessing, die Herstellung von Kosmetik, Papier oder Nahrung, Arzneimittelherstellung und anderes umfassen. Solche Bereiche können sich auch auf den Maschinenbau, insbesondere auf den Bereich von Haushalts- oder Küchengeräten erstrecken.

Erfindungsgemäß wird ferner eine Überwachungsvorrichtung vorgeschlagen mit wenigstens einem Ortsangabesensor (z. B. GPS) und/oder Bewegungssensor, welcher jede beliebige Kombination von Merkmalen aufweist, die hierin für den Bewegungssensor offenbart sind. In diesen Ausführungsformen ist der Bewegungssensor nicht Teil des erfindungsgemäßen Pumpenantriebs, sondern kann Teil jeder anderen Vorrichtung sein, die der Überwachung dienen kann.

Eine solche Überwachungsvorrichtung kann lösbar mit einer beliebigen Vorrichtung verbunden werden oder sein, etwa mit einer Behandlungsvorrichtung wie einer Vorrichtung zur Zellbehandlung, Desinfektion, Spülung, parenteralen Ernährung und dergleichen, insbesondere einer Blutbehandlungsvorrichtung, ganz insbesondere einer Hämodialysevorrichtung, Hämofiltrationsvorrichtung oder einer Hämodiafiltrationsvorrichtung, insbesondere einer Vorrichtung für die chronische Nierenersatztherapie oder für die kontinuierliche Nierenersatztherapie (CRRT = continuous renal replacement therapy).

Eine solche Überwachungsvorrichtung steht in einigen Ausführungsformen nicht mit der Steuerung oder Regelung der mit ihr verbundenen Vorrichtung in Signalkommunikation. Insbesondere liegt keine hierzu erforderliche Konfiguration vor.

Eine solche Überwachungsvorrichtung steht in einigen Ausführungsformen in Signalkommunikation mit wenigstens einem externen Gerät, wie hierin definiert. Die Überwachungsvorrichtung und/oder das externe Gerät sind konfiguriert, um den Nutzer, vorzugweise automatisch, über Ergebnisse, die die mittels der Überwachungsvorrichtung überwachte Vorrichtung, z. B. die Behandlungsvorrichtung, betreffen, zu informieren. Solche Ergebnisse können Erschütterungen der Vorrichtung, Kollisionen, ihren Zustand ("steht die Vorrichtung still oder ist sie in Bewegung"), ihren Standort ("wo steht das Gerät") usw., betreffen.

Eine solche Überwachungsvorrichtung ist in einigen Ausführungsformen lösbar mit der Behandlungsvorrichtung verbunden. Die Verbindung kann mittels Magnetvorrichtungen, Klemmvorrichtungen, Halterungen, Rasteinrichtungen oder dergleichen erfolgen. Entsprechende Einrichtungen hierzu können vorgesehen sein.

In manchen Ausführungsformen ist die Spannungsquelle innerhalb des Gehäuses des Pumpenantriebs vorgesehen.

In einigen Ausführungsformen ist die Pumpe keine Kolbenpumpe.

In manchen Ausführungsformen weist die Pumpe oder der Pumpenantrieb keinen Kolben auf, der angeordnet ist, um im Gehäuse axial zu oszillieren.

In einigen Ausführungsformen weist die Pumpe kein Rückschlagventil auf.

In manchen Ausführungsformen ist der Pumpenantrieb nicht ausgestaltet, um vom gepumpten Fluid zwischen einem Gehäuseeinlass und einem Gehäuseauslass durchströmt zu werden.

Manche oder alle erfindungsgemäßen Ausführungsformen können einen, mehrere oder alle der oben und/oder im Folgenden genannten Vorteile aufweisen.

Ein Vorteil der vorliegenden Erfindung besteht darin, dass von der Benutzung der akku-betriebenen Pumpe keine Gefahr für den Patienten, die sich aus der Leitung von Strom ergeben könnte, ausgeht.

Ein weiterer Vorteil der vorliegenden Erfindung besteht darin, dass mittels der erfindungsgemäßen Pumpe bzw. ihrer Steuerelektronik ein Rückschlagventil eingespart werden kann.

Ferner ist es von Vorteil, dass die erfindungsgemäße Pumpe vorgesehen sein kann, zwar manuell zusammengesetzt zu werden, danach aber automatisch einer Programmierung zu folgen. So können das Starten der Pumparbeit, das Anpassen von Drehzahlen und andere Schritte, die hierin beschrieben sind, sowie weitere Schritte und Funktionen automatisch erfolgen. Der Nutzer ist damit in seiner Arbeit entlastet.

Zudem erfordert die vorliegende Erfindung in vielen Ausführungsformen keinen Eingriff in die Steuerung oder Regelung der Behandlungsvorrichtung und kein manuelles Starten der Pumpe, um den Effluentbeutel zu entleeren.

Ein weiterer Vorteil besteht darin, unerwünschte Bewegungen der Behandlungsvorrichtung im Raum mittels des Zusammenwirkens von Bewegungssensor, Wireless-Modul und externem Gerät anzeigen zu lassen.

Im Folgenden wird die vorliegende Erfindung unter Bezugnahme auf die beigefügten Figuren rein exemplarisch beschrieben. In ihnen bezeichnen selbe Bezugszeichen gleiche oder selbe Komponenten. Es gilt:
- **Fig. 1**: zeigt in vereinfachter Darstellung eine erfindungsgemäße medizinische Behandlungsvorrichtung, hier exemplarisch eine Blutbehandlungsvorrichtung mit einem extrakorporalen Blutkreislauf, in einer ersten Ausführungsform;
- **Fig. 2**: zeigt in vereinfachter Darstellung eine erfindungsgemäße Pumpe, eingebunden in ein Abflussschlauchsystem der Blutbehandlungsvorrichtung der Fig. 1;
- **Fig. 3**: zeigt in vereinfachter Darstellung einen erfindungsgemäßen Pumpenantrieb (oben) und einen Pumpenabschnitt (unten), die gemeinsam eine erfindungsgemäße Pumpe ergeben, in einer ersten Ausführungsform in teilweiser Schnittdarstellung; und
- **Fig. 4**: zeigt in vereinfachter Darstellung den Pumpenantrieb der Fig. 3 (oben) mit einer erfindungsgemäßen Induktionsladestation (unten).

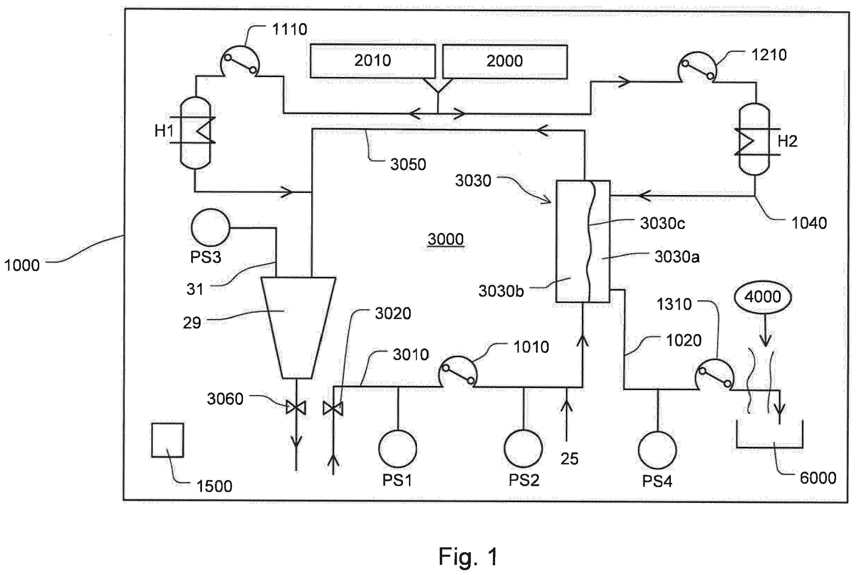

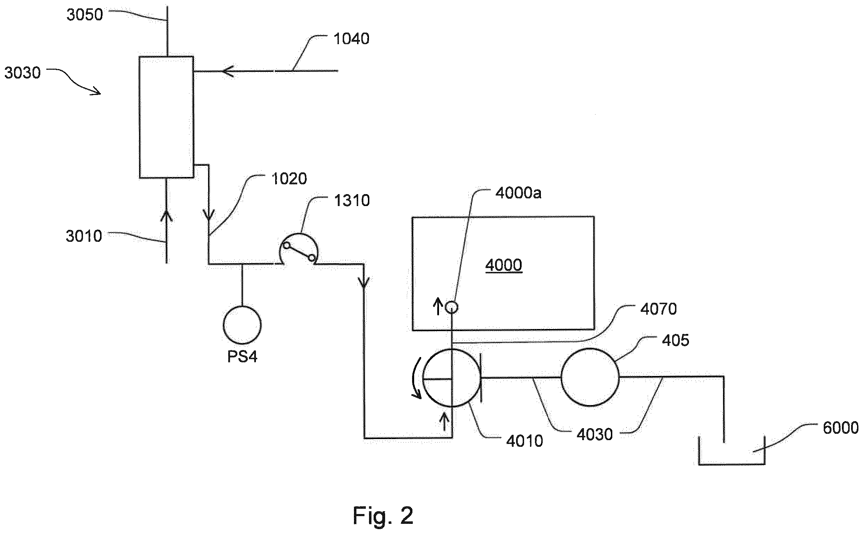

**Fig. 1** zeigt in stark vereinfachter Darstellung eine erfindungsgemäße Blutbehandlungsvorrichtung 1000 als Beispiel für eine erfindungsgemäße medizinische Behandlungsvorrichtung, verbunden mit einem extrakorporalen Blutkreislauf 3000 als Beispiel eines erfindungsgemäßen Schlauchsatzes und einem nur angedeuteten erfindungsgemäßen Abflussschlauchsystem mit einem Effluentbeutel 4000. Abflussschlauchsystem und Effluentbeutel 4000 gehen aus Fig. 2 hervor. Die Blutbehandlungsvorrichtung 1000 der Fig. 1 weist ferner eine erfindungsgemäße Pumpe 405 auf oder ist damit verbunden. Gezeigt ist diese jedoch ebenfalls erst in den folgenden Figuren.

Der extrakorporale Blutkreislauf 3000 weist eine erste Leitung 3010, hier in Form eines arteriellen Leitungsabschnitts, auf.

Die erste Leitung 3010 steht in Fluidverbindung mit einer Blutbehandlungseinrichtung, hier exemplarisch ein Blutfilter oder Dialysator 3030. Der Blutfilter 3030 weist eine Dialysierflüssigkeitskammer 3030a und eine Blutkammer 3030b auf, welche durch eine zumeist semi-permeable Membran 3030c voneinander getrennt sind.

Der extrakorporale Blutkreislauf 3000 weist ferner wenigstens eine zweite Leitung 3050, hier in Form eines venösen Leitungsabschnitts, auf. Sowohl die erste Leitung 3010 als auch die zweite Leitung 3050 können zur Verbindung mit dem Gefäßsystem des nicht dargestellten Patienten dienen.

Die erste Leitung 3010 ist optional mit einer (ersten) Schlauchklemme 3020 zum Sperren oder Schließen der Leitung 3010 verbunden. Die zweite Leitung 3050 ist optional mit einer (zweiten) Schlauchklemme 3060 zum Sperren oder Schließen der Leitung 3050 verbunden.

Die in Fig. 1 nur durch einige ihrer Einrichtungen und schematisch repräsentierte Blutbehandlungsvorrichtung 1000 weist eine Blutpumpe 1010 auf. Die Blutpumpe 1010 fördert während der Behandlung des Patienten Blut durch Abschnitte des extrakorporalen Blutkreislaufs 3000 und in Richtung zum Blutfilter oder Dialysator 3030, wie die kleinen Pfeilspitzen, welche in jeder der Fig. 1 und 2 allgemein die Strömungsrichtung angeben, zeigen.

Mittels einer Pumpe 1210 für Dialysierflüssigkeit, die als Rollenpumpe oder als anderweitig okkludierende Pumpe ausgestaltet sein kann, wird frische Dialysierflüssigkeit aus einer Quelle 2000 entlang der Dialysierflüssigkeitszulaufleitung 1040 in die Dialysierflüssigkeitskammer 3030a gepumpt. Die Dialysierflüssigkeit verlässt die Dialysierflüssigkeitskammer 3030a als Dialysat, ggf. angereichert durch Filtrat, in Richtung des Ausgusses 6000 und wird hierin als Effluent bezeichnet.

Die Quelle 2000 kann beispielsweise ein Beutel oder ein Container sein. Die Quelle 2000 kann ferner eine Fluidleitung sein, aus der online und/oder kontinuierlich erzeugte oder gemischte Flüssigkeit bereitgestellt wird, z. B. ein Hydraulikausgang oder -anschluss der Blutbehandlungsvorrichtung 1000.

Eine weitere Quelle 2010 mit Substituat kann optional vorgesehen sein. Sie kann der Quelle 2000 entsprechen oder eine eigene Quelle sein.

Rechts unten ist in Fig. 1 angedeutet, wo das Abflussschlauchsystem (mit dem Effluentbeutel 4000) mit der Blutbehandlungsvorrichtung 1000 verbunden ist.

Neben der vorgenannten Blutpumpe 1010 weist die in Fig. 1 gezeigte Anordnung ferner rein optional eine Reihe weiterer, jeweils optionaler Pumpen, nämlich die Pumpe 1110 für Substituat, zum Fördern von Substituat, vorzugsweise durch eine optionale Beutelheizung H1 hindurch, die Pumpe 1210 für Dialysierflüssigkeit, und die Pumpe 1310 für das Effluent auf.

Die Pumpe 1210 ist vorgesehen, um Dialysierflüssigkeit aus einer Quelle 2000, beispielsweise einem Beutel, heraus und über eine optional vorhandene Beutelheizung mit einem Beutel H2 dem Blutfilter 3030 mittels einer Dialysierflüssigkeitszulaufleitung 1040 zuzuführen.

Die auf diese Weise zugeführte Dialysierflüssigkeit tritt über eine Dialysatablaufleitung 1020, unterstützt durch die Pumpe 1310, wieder aus dem Blutfilter 3030 und kann verworfen werden.

Stromauf der Blutpumpe 1010 ist ein optionaler arterieller Sensor PS1 vorgesehen. Während einer Behandlung des Patienten misst er den Druck in der arteriellen Leitung.

Stromab der Blutpumpe 1010, jedoch stromauf des Blutfilters 3030 und, falls vorgesehen, einer Zugabestelle 25 für Heparin, ist ein weiterer, optionaler Drucksensor PS2 vorgesehen. Er misst den Druck stromauf des Blutfilters 3030 ("prä-Hämofilter").

Ein wiederum weiterer Drucksensor kann als PS4 stromab des Blutfilters 3030, jedoch vorzugsweise stromauf der Pumpe 1310 in der Dialysatablaufleitung 1020 zum Messen des Filtratdrucks des Blutfilters 3030 vorgesehen sein.

Blut, das den Blutfilter 3030 verlässt, durchströmt eine optionale venöse Blutkammer 29, welche eine Entlüftungseinrichtung 31 aufweisen und mit einem weiteren Drucksensor PS3 in Fluidverbindung stehen kann.

Eine Steuer- oder Regelvorrichtung 1500 zum Steuern oder Regeln der Blutbehandlungsvorrichtung 1000 kann vorgesehen und mit allen o. g. Komponenten der Blutbehandlungsvorrichtung 1000 in Signal- und/oder Steuerverbindung stehen.

**Fig. 2** zeigt in vereinfachter Darstellung ein erfindungsgemäßes Abflussschlauchsystem mit einem erfindungsgemäßen Effluentbeutel 4000 in einem Moment, während welchem dem Effluentbeutel 4000 Effluent zugeführt wird.

Eine optionale Umschalteinrichtung 4010, hier exemplarisch ausgestaltet als Drei-Wege-Hahn 4010, ist zwischen der Pumpe 1310 für das Effluent und dem Effluentbeutel 4000, jedenfalls aber stromauf zu diesem, in der Dialysatablaufleitung 1020 angeordnet. Zwischen Umschalteinrichtung 4010 und Effluentbeutel 4000 kann eine Verbindungsleitung 4070 vorgesehen sein. Über die Dialysatablaufleitung 1020 läuft Dialysat (und/oder Effluent) aus dem Blutfilter 3030 ab. Sie dient zugleich aber auch als Effluentzulaufleitung, da sie dem Effluentbeutel 4000 Effluent zuleitet.

Wie in Fig. 2 gezeigt ist, verbindet der Drei-Wege-Hahn 4010 in seiner in Fig. 2 gezeigten Stellung die Dialysatablaufleitung 1020 mit der Effluenteinlassöffnung, welche auch die Effluentauslassöffnung ist, daher kurz: Effluentöffnung 4000a, des Effluentbeutels 4000 fluidisch.

Gesperrt ist in dieser ersten Stellung die ebenfalls mit dem Drei-Wege-Hahn 4010 verbundene Effluentablaufleitung 4030, welche ihrerseits, direkt oder indirekt, mit dem Ausguss 6000 verbunden ist.

Die Effluentablaufleitung 4030 kann Teil eines Schlauchsatzes sein.

In der in Fig. 2 gezeigten ersten Stellung kann Effluent aus der Dialysatablaufleitung 1020 über den Drei-Wege-Hahn 4010 hinweg in den Effluentbeutel 4000 gelangen, nicht jedoch in die Effluentablaufleitung 4030. Der Drei-Wege-Hahn 4010 kann aufgrund seiner Ausgestaltung aus elektrisch isolierendem Material sein und/oder an den Stellen, an welchen er flüssigkeitsführend ist, eine elektrische Isolierung bewirken.

In die Effluentablaufleitung 4030 ist eine erfindungsgemäße Pumpe 405 eingebunden. Die Effluentablaufleitung 4030 mündet stromab der Pumpe 405 in den Ausguss 6000.

Die Pumpe 405 ist stromab des Effluentbeutels 4000 aber stromauf des Ausgusses 6000 angeordnet.

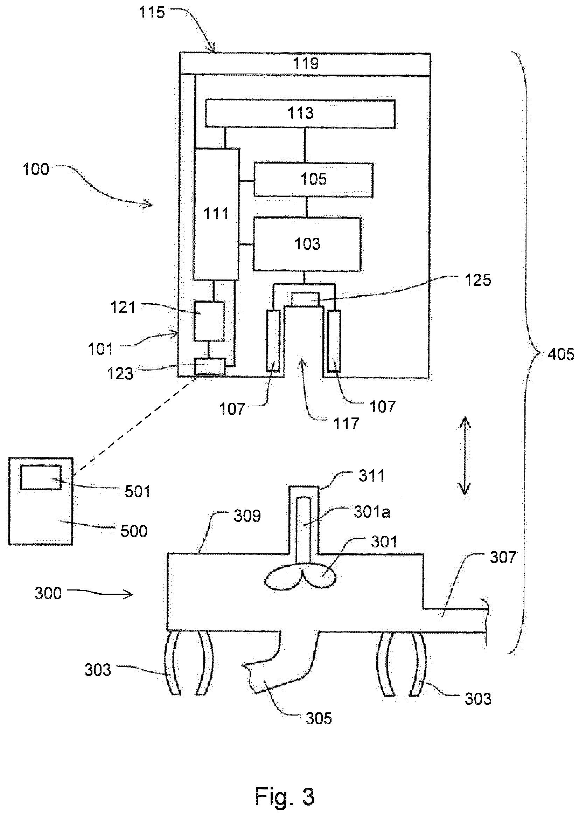

Die Pumpe 405, welche wenigstens einen Pumpenantrieb 100, wie in Fig. 3 gezeigt, aufweist, ist in der Stellung der Fig. 2 nicht in Betrieb ("OFF"), da die Effluentablaufleitung 4030 kein Effluent führt, welche mittels der Pumpe 405 in den Ausguss 6000 verworfen werden könnte.

**Fig. 3** zeigt in stark vereinfachter Darstellung einen erfindungsgemäßen medizinischer Pumpenantrieb 100 der Pumpe 405 der Fig. 2 mit einem Pumpengehäuse 101. Im Pumpengehäuse 101 sind ein Pumpenmotor 103, eine wiederladbare Spannungsquelle oder ein Akku 105, ein Magnetabschnitt 107 zum, insbesondere kontaktlosen, Antreiben eines Pumpenrotors 301, eine Steuer- oder Regelelektronik 111 zum Steuern oder Regeln des Ladens des Akkus sowie weiterer Funktionen des Pumpenantriebs 100 und eine Induktionsladespule 113 zum Laden des Akkus 105 mittels Induktion untergebracht. Das Pumpengehäuse 101 kann einteilig oder mehrteilig sein.

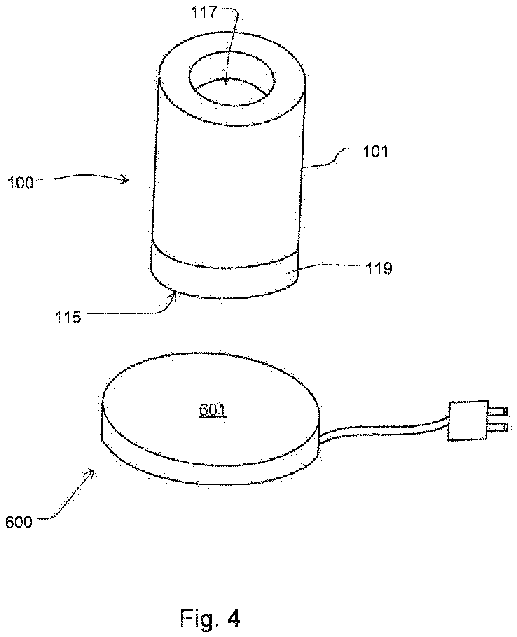

Das Pumpengehäuse 101, welches hier rein exemplarisch zylindrischer Gestalt ist, weist an einem seiner Enden oder Endbereiche (in Fig. 3 am oberen Ende des Pumpengehäuses 101) ein Aufstellabschnitt 115 in Gestalt einer optional ebenen Fläche auf. Mittels des Aufstellabschnitts 115 kann der Pumpenantrieb 100 (bezogen auf seine Darstellung in Fig. 3) sozusagen auf den Kopf gestellt aufgestellt werden, ein Zustand, der in Fig. 4 gezeigt ist. Aus offensichtlichen Gründen liegt die Induktionsladespule 113 vergleichsweise nahe am Aufstellabschnitt 115, liegt diesem optional sogar körperlich an.

Das Pumpengehäuse 101 weist ferner einen Verbindungsabschnitt 117 auf, welcher dem Ankoppeln des in Fig. 3 oben gezeigten Pumpengehäuses 100 mit dem in Fig. 3 unten gezeigten Pumpenabschnitt 300 dient.

Unter einem Verbinden ist in der hier gezeigten und weiteren Ausführungsformen ein Zusammenbringen von Pumpenantrieb 100, welche den Pumpenmotor 103 für einen Pumpenrotor 301 aufweist, und Pumpenabschnitt 300, welcher den Pumpenrotor 301 aufweist, zu verstehen. Das Verbinden ist hier also als ein Zusammenbringen derart zu verstehen, dass im Ergebnis eine funktionsfähige Pumpe 405 entsteht.

Das Verbinden umfasst in einigen Ausführungsformen, etwa der in Fig. 3 und Fig. 4 gezeigten, kein mechanisches Fixieren des Pumpengehäuses 101 oder des Pumpenantriebs 100 am Pumpenabschnitt 300.

Das Verbinden umfasst in einigen Ausführungsformen, hierunter zählt jene der Fig. 3 oder Fig. 4, keine mechanischen Einrichtungen zum Halten des Pumpenantriebs 100, am Pumpenabschnitt 300, insbesondere keine Klauen oder Claws, von denen mehrere um den Umfang verteilt sein könnten. Auch umfassen diese Ausführungsformen keine, z.B. horizontalen, Platten oder Vorsprünge, die in anderen Elementen, etwa Klauen oder Klemmen, die zum Herstellen einer Verbindung dienen, aufgenommen werden würden.

Im Beispiel der Fig. 3 und 4 sowie weiteren, beliebigen Ausführungsformen hält der Pumpenantrieb 100 auf dem Pumpenabschnitt 300 bzw. der Ladeplattform 600 mittels Schwerkraft, ggf. unterstützt durch magnetische Anziehung zwischen Pumpenantrieb 100 auf dem Pumpenabschnitt 300.

Ein seitliches Verrutschen des Pumpenantriebs 100 bezogen auf den Pumpenabschnitt 300, oder umgekehrt, kann auf einfache Weise dadurch verhindert werden, dass der Verbindungsabschnitt 117 als eine Kavität oder Sacköffnung ausgestaltet ist wie im Beispiel der Fig. 3, in welche eine Erhebung 311 des Pumpenabschnitts 300 eingesteckt werden kann, insbesondere durch eine einfache Bewegung in der Verbindungsrichtung, die in Fig. 3 mit einem Doppelpfeil gezeigt ist, und der kein Befestigen, etwa mittels eines Hebels, durch Verdrehen des Pumpenantriebs 100 relativ zum Pumpenabschnitt 300 zum gegenseitigen Verriegeln dieser beiden Bauteile, usw. vorausgeht oder folgt. Alternativ oder ergänzend können Verrutschsicherungen vorgesehen sein, etwa in Form von Rändern, Verhakungen, Eingriffsstiften, usw., die, um in Eingriff zu kommen, vorzugsweise jeweils keiner Betätigung und/oder keines Handgriffs des Nutzers bedürfen.

Wie in der angedeuteten Schnittdarstellung der Fig. 3 zu erkennen, ist der Magnetabschnitt 107 innerhalb des Pumpengehäuses 101 um den dem Verbindungsabschnitt 117 herum rotierbar gelagert.

Wie in der angedeuteten Schnittdarstellung der Fig. 3 weiter zu erkennen ist, ist der Pumpenrotor 301 innerhalb der Erhebung 311 rotierbar gelagert.

Das Pumpengehäuse 101 weist ferner wenigstens eine von außerhalb des Pumpengehäuses 101 sichtbare Leuchteinrichtung 119 auf. Sie kann mittels der Steuerelektronik 111 angesteuert werden, um mittels Licht Zustände oder Funktionen des Pumpenantriebs 100 oder seiner Bauteile für den Benutzer des Pumpenantriebs 100 oder der Pumpe 405 zu signalisieren.

Die Leuchteinrichtung 119 kann ein Ring, insbesondere mit einer oder mehreren LEDs, oder ein LED-Farbring sein.

Auch zu diesem Zweck kann der Pumpenantrieb 100 ein Wireless-Modul zur Daten- oder Signalübertragung aufweisen. Eine solche Übertragung kann auf ein externes Gerät 500 erfolgen. Mittels eines Displays 501 des externen Geräts 500 können dem Nutzer Informationen den Pumpenantrieb 100 oder die Pumpe 405 betreffend übermittelt werden, etwa den Modus angeben, den Ladezustand des Akkus, usw.

Der Pumpenantrieb 100 kann optional ferner einen Bewegungssensor 123 aufweisen, der darüber informiert, ob oder dass der Pumpenantrieb 100 bewegt wurde. Eine solche Bewegung kann die Steuerelektronik 111 veranlassen, von einem ersten Zustand (etwa einem stromsparenden sleep-Modus) in einen zweiten Zustand (etwa einen awake-Modus, in dem die Leuchteinrichtung 119 aktiviert, umgeschaltet oder angeschaltet wird) überzugehen.

Der Pumpenabschnitt 300, der den Pumpenrotor 301 aufweist, kann eine Einrichtung zum lösbaren Befestigen des Pumpenabschnitts 300 an einem Abschnitt der in Fig. 1 schematisch dargestellten Blutbehandlungsvorrichtung 1000 oder einer anderen medizinischen Behandlungsvorrichtung aufweisen.

Die Einrichtung zum lösbaren Befestigen kann eine Clipeinrichtung 303 sein, die ein- oder mehrteilig ist.

Der Pumpenabschnitt 300 kann Teil eines Disposables sein.

Dieses Disposable kann ferner Schlauchabschnitte der Effluentablaufleitung 4030 aufweisen, von welchen hier exemplarisch nur ein Fluideinlass 305 und ein Fluidauslass 307 bzw. deren Konnektoren gezeigt sind, mittels welcher zu pumpende Flüssigkeit in ein Gehäuse 309 des Pumpenabschnitts 300 bzw. aus diesem herausströmt.

Fig. 3 zeigt den Pumpenantrieb 100 und den Pumpenabschnitt 300 kurz vor ihrem Verbinden zur Pumpe 405. Sind sie zur Pumpe 405 verbunden, so behalten sie ihre Ausrichtung in einer Gebrauchsstellung der Pumpe 405 insoweit bei, als der Verbindungsabschnitt 117 das untere Ende des Pumpenantriebs 100 darstellt und die Erhebung 311 das obere Ende des Pumpenabschnitts 300. Verallgemeinert ausgedrückt, wird der Pumpenantrieb 100 von oben auf den Pumpenabschnitt 300 zu seinem Verbinden zu einer Pumpe 405 aufgesetzt, nicht umgekehrt. Auch während des Gebrauchs der Pumpe 405 liegt der Pumpenantrieb 100 oben, der Pumpenabschnitt 300 unten. Nach Beendigung des Einsatzes der Pumpe 405 wird der Pumpenantrieb 100 nach oben vom Pumpenabschnitt 300 abgehoben.

Im Folgenden wird ein typischer Gebrauch des erfindungsgemäßen Pumpenantriebs 100 beschrieben.

Wird der Pumpenantrieb 100 zum Gebrauch von der Induktionsladestation 600, die nicht auf ein Laden mittels Induktion beschränkt ist, sondern alternativ auch von anderen Verfahren Gebrauch machen kann, genommen, so detektiert der (optional Lowpower-) Bewegungssensor 123 Aktivität und weckt die Steuerelektronik 111 auf. Als Folge wird optional der Ladezustand des Akkus 105 mittels des Farbrings 119, z. B. in einer ersten Farbe, angezeigt.

Alternativ oder ergänzend wird, z. B. durch eine andere Farbe als die erste, angezeigt, dass eine Bewegung erkannt wurde.

Zum Zusammensetzen der Pumpe 405 durch Aufsetzen des Pumpenantriebs 100 auf dem Pumpenabschnitt 300 wird der Antrieb um 180° um eine Horizontale gedreht.

Die Pumpenantriebskavität oder der Verbindungsabschnitt 117 zeigt nun nach unten und kann entsprechend auf die Erhebung 311 des Pumpenabschnitts 300 aufgesetzt werden, welcher den Pumpenrotor 301 beinhaltet und nach oben zeigend ausgerichtet ist.

Der Pumpenrotor 301, der einen Magnetabschnitt 301a aufweist, welcher ohne die Wirkung und Nähe des Pumpantriebs 100 normalerweise schwerkraftbedingt nach unten weiter in das Innere des Pumpenabschnitts 300 fallen würde, wird bei Annäherung des Pumpantriebs 100 von dessen Eisenankern ("Statoren") nach oben magnetisch angezogen. Der Pumpantrieb 100 wird - für den Benutzer spürbar - entsprechend nach unten gezogen. Die formschlüssigen Teile finden scheinbar selbstständig zueinander und gelangen in eine sehr klar definierte Position in die Betriebsstellung der Pumpe 405, in welcher diese funktionsfähig ist.

Die - bereits durch die initiale Bewegung des Pumpantriebs 100, wenn dieser von der Induktionsladestation 600 abgehoben wird - aufgeweckte Steuerelektronik 111, überwacht und erkennt den in den Verbindungsabschnitt 117, der eine Gehäusekavität sein kann, eingeführten Magnetabschnitt 301a. Die Erkennung kann z. B. mittels optionaler Hallsensoren 125 oder anderen Einrichtungen erfolgen und startet automatisch ein abgespeichertes Programm: der Magnetabschnitt 301a kommt in einen Schwebezustand aufgrund des Aktivierens der Lageregelung, und der Magnetabschnitt 301a wird auf eine definierte Drehzahl beschleunigt (Antrieb aktiv).

Durch die oberhalb der Pumpe angeordnete Flüssigkeit im Effluentbeutel 4000 ist sichergestellt, dass Flüssigkeit im Pumpenkopf zur Verfügung steht und sofort große Mengen Flüssigkeit gefördert werden können. Die Leuchteinrichtung 119, hier der Farbring, signalisiert den Pumpvorgang mit einem (z. B. blauen), optional sich drehenden, Farbring oder umlaufenden Lichtschein.

Ein "Farbring", so wie hierin verwendet, kann ein Ring von einzelnen Leuchtmitteln, etwa eine Reihe von benachbarten LEDs, sein.

Wenn die Pumpe 405 den Flüssigkeitsvorrat des Effluentbeutels 4000 leergepumpt hat und Luft ansaugt oder einen Unterdruck erzeugt (z. B. bei kollabierter Saugleitung), erkennt die Steuerelektronik 111 optional eine Änderung der Antriebs- oder der Lagerströme. Die Antriebsströme resultieren aus der zum Erhalten der Drehzahl erforderlichen Leistung. Ändert sich der Widerstand der Flüssigkeit (z. B. durch Luftblasen, Unterdruck, Dichte) muss der Antriebsstrom optional angepasst werden, um die Drehzahl konstant halten zu können. Dieses Überwachen wird optional genutzt, um eine "Beutel-leer" Detektion einzuleiten: die maximale bzw. voreingestellte Drehzahl wird reduziert. Es kann vorteilhaft sein, eine Drehzahl zu wählen, welche gerade ausreicht, die Flüssigkeit am Zurückströmen zu hindern. Es stellt sich also vorzugsweise ein Gleichgewicht zwischen der nach oben gepumpten Flüssigkeit und der nach unten strömenden - der Schwerkraft folgenden - Flüssigkeit ein. Durch diese Maßnahme wird z. B. ein Rückschlagventil überflüssig. Diese optionale Sperrfunktion kann wieder durch ein entsprechendes Signal der Leuchteinrichtung bzw. des Farbrings 119 angezeigt werden (z. B. ändert sich ein blauer, sich drehender Farbring (=Pumpvorgang) in einen blau pulsierenden Farbring, was die Sperrfunktion anzeigt, die die Funktion eines Rückschlagventils haben kann).

Nach dem Pumpvorgang wird der Pumpenantrieb 100 wieder um 180° gedreht und mit seiner Induktionsspule 113 nach unten auf die Induktionsladestation 600 gestellt. Durch ein optionales, z. B. gelegentliches, Lichtsignal der Leuchteinrichtung bzw. des Farbrings 119 wird der Ladevorgang angezeigt. Wenn der Akku 105 wieder zu z. B. 100% geladen ist, geht der Pumpenantrieb 100 bzw. seine Steuerelektronik 111 zurück in den ersten Zustand, z. B. den *LowPower-Modus.*

Die Wireless-Schnittstelle 121 kann konfiguriert sein, um z. B. per bluetooth-Protokoll zu übertragen.

Das Übertragen kann z. B. an einem Empfänger der Blutbehandlungsvorrichtung, deren Display eine entsprechende Anzeige machen kann, oder welche eine andere Routine auslösen kann, erfolgen. Das Übertragen kann alternativ oder ergänzend auf ein externes Gerät 500, wie z. B. ein Smartphone oder ein anderes Handgerät oder mobiles Gerät, erfolgen.

Ist die Pumpe 405 und/oder die Induktionsladestation 600 direkt oder kraftschlüssig mit der Blutbehandlungsvorrichtung verbunden, so ist es möglich, die Daten des Bewegungssensors 123 per Wireless-Schnittstelle 121 auf das externe Gerät 500 und damit an den Anwender zu schicken. Hierdurch könnte also, ohne irgendeinen Eingriff in die Geräteelektronik oder Software der Blutbehandlungsvorrichtung 1000 eine Bewegungswarnung der Blutbehandlungsvorrichtung 1000 erfolgen. Bei waagebasierten Geräten würde das eine wichtige Information für den Anwender bedeuten, da das Bilanziersystem der Blutbehandlungsvorrichtung 1000 bei Erschütterungen eine entsprechende Fehlerkaskade auslöst. Der Anwender hat also schon im Vorfeld eine Möglichkeit nach der Blutbehandlungsvorrichtung 1000, etwa der Dialysemaschine, zu schauen und eventuell leicht behebbare Fehler frühzeitig zu eliminieren, die Ursache für die Erschütterung sind. Solche Ursachen umfassen eine Kollision der Blutbehandlungsvorrichtung mit anderem Gerät, einen pulsierenden Schlauch, der gegen die Blutbehandlungsvorrichtung schlägt, usw. Von Vorteil ist hierbei insbesondere, dass eine Mitteilung an den Nutzer ergehen kann, die ein möglichst schnelles Reagieren erlaubt, vorzugsweise noch bevor die Maschine automatisch Maßnahmen einleitet, etwa weil die Bilanzierwaagen auf die Erschütterungen hin unbemerkt ihre Tätigkeit einstellen. Nach Auswertung des Waagenfehlers kann die Bilanziereinrichtung beispielsweise durch den Anwender ausgeschaltet werden; er ist sich dessen anschließend bewusst.

**Fig. 4** zeigt in ihrem oberen Teil in vereinfachter Darstellung den erfindungsgemäßen Pumpenantrieb 100 in leichter Perspektive von oben. Sie zeigt in ihrem unteren Teil eine erfindungsgemäße Induktionsladestation 600.

Zum Laden des Akkus 105 des Pumpenantriebs 100 wird dieser von oben und mit seinem Ende, welchem die in Fig. 4 nicht gezeigte Induktionsspule 113 zugeordnet ist, auf eine Aufstellfläche 601 der Induktionsladestation 600 aufgesetzt. Dabei wird eine waagerechte oder im Wesentlich waagerechte Aufstellung oder Ausrichtung der Induktionsladestation 600 bzw. seiner Aufstellfläche 601 vorausgesetzt. In der dann erreichten Position wird er im Beispiel der Fig. 4 nicht weiter befestigt oder verbunden. Er hält aufgrund seines Gewichts auf der Aufstellfläche 601. Halterungen oder Befestigungen können jedoch vorgesehen sein.

Die vorstehenden Ausführungen, die insbesondere mit Blick auf die Figuren gemacht sind und eine Blutbehandlungsvorrichtung betreffen, sind nicht auf eine medizinische Vorrichtung zur Behandlung von Blut beschränkt zu verstehen. Auf andere medizinische Vorrichtungen, welche kein Blut behandeln, gleichwohl aber ein medizinisches Fluid mittels Pumpe fördern, trifft die vorstehende Offenbarung unbeschränkt ebenfalls zu.

### Bezugszeichenliste

- 25: Zugabestelle für Heparin (optional)
- 29: venöse Blutkammer
- 31: Entlüftungseinrichtung

- 100: Medizinischer Pumpenantrieb, auch: Pumpenantrieb
- 101: Pumpengehäuse
- 103: Pumpenmotor
- 105: wiederladbare Spannungsquelle oder Akku
- 107: Magnetabschnitt
- 111: Steuerelektronik
- 113: Induktionsladespule
- 115: Aufstellabschnitt
- 117: Verbindungsabschnitt
- 119: Leuchteinrichtung
- 121: Wireless-Modul oder -Schnittstelle
- 123: Bewegungssensor
- 125: Hallsensor

- 300: Pumpenabschnitt
- 301: Pumpenrotor
- 301a: Magnetabschnitt
- 303: Clipeinrichtung
- 305: Fluideinlass
- 307: Fluidauslass
- 309: Gehäuse
- 311: Erhebung

- 405: Pumpe

- 500: externes Gerät
- 501: Display

- 600: Induktive Ladeplattform
- 601: Aufstellfläche

- 1000: Blutbehandlungsvorrichtung
- 1010: Blutpumpe
- 1020: Dialysatablaufleitung, Effluentzulaufleitung
- 1040: Dialysierflüssigkeitszulaufleitung
- 1110: Pumpe für Substituat
- 1210: Pumpe für Dialysierflüssigkeit
- 1310: Pumpe für Dialysat oder Effluent
- 1500: Steuer- oder Regelvorrichtung

- 2000: Quelle mit Dialysierflüssigkeit
- 2010: Quelle mit Substituat, optional

- 3000: extrakorporaler Blutkreislauf
- 3010: erste Leitung (arterieller Leitungsabschnitt)
- 3020: (erste) Schlauchklemme
- 3030: Blutfilter oder Dialysator
- 3030a: Dialysierflüssigkeitskammer
- 3030b: Blutkammer
- 3030c: semi-permeable Membran
- 3050: zweite Leitung (venöser Leitungsabschnitt)
- 3060: (zweite) Schlauchklemme

- 4000: Effluentbeutel
- 4000a: Effluenteinlass- oder -auslassöffnung; Effluentöffnung
- 4010: Drei-Wege-Hahn, Umschalteinrichtung

- 4030: Effluentablaufleitung, Schlauchsatz
- 4070: Verbindungsleitung

- 6000: Ausguss

- H2: Beutelheizung mit Beutel (Dialysierflüssigkeit)
- H1: Beutelheizung mit Beutel (Substituat)

- PS1, PS2: arterieller Drucksensor (optional)
- PS3: venöser Drucksensor (optional)
- PS4: Drucksensor zum Messen des Filtratdrucks

## Patentansprüche

1. Medizinischer Pumpenantrieb (100), mit wenigstens :
- wenigstens einem Gehäuse (101) und einem im Gehäuse (101) vorgesehenen Pumpenmotor (103);
- wenigstens einer wiederladbaren Spannungsquelle zum Speichern von elektrischer Energie, oder einem Akku (105), für die Spannungsversorgung des Pumpenmotors (103);
- wenigstens einem Magnetabschnitt (107) zum magnetischen Ankoppeln und/oder magnetischen Antreiben eines magnetisch antreibbaren Pumpenrotors (301); und
- wenigstens einer Steuerelektronik (111), welche programmiert ist, den Magnetabschnitt (107) des Pumpenantriebs (100) zu aktivieren;
**dadurch gekennzeichnet, dass** die Steuerelektronik (111) mit einem Bewegungssensor (123) ausgestaltet oder verbunden ist, welcher zum Erkennen einer Position oder Bewegung des medizinischen Pumpenantriebs (100) im Raum konfiguriert ist.

2. Pumpenantrieb (100) nach Anspruch 1, mit einer Induktionsladespule (113) zum Laden der Spannungsquelle oder des Akkus (105).

3. Pumpenantrieb (100) nach einem der vorangegangenen Ansprüche, wobei das Gehäuse (101) aufweist:
- einen Aufstellabschnitt (115) zum Aufstellen des Pumpenantriebs (100) auf einer Aufstellfläche; und
- einen Verbindungsabschnitt (117) zum Verbinden des Pumpenantriebs (100) mit einem Pumpenabschnitt (300), welcher einen Pumpenrotor (301) aufweist;
wobei der Aufstellabschnitt (115) und der Verbindungsabschnitt (117) an gegenüberliegenden Enden des Gehäuses (101) liegen oder diesen zugeordnet sind.

4. Pumpenantrieb (100) nach einem der vorangegangenen Ansprüche, mit einer, insbesondere farbigen Leuchteinrichtung (119), insbesondere ausgestaltet als Ring, insbesondere als LED oder LED-Farbring, und/oder insbesondere im oder am Pumpengehäuse (101) ausgestaltet.

5. Pumpenantrieb (100) nach einem der vorangegangenen Ansprüche, wobei die Steuerelektronik (111) mit einem Wireless-Modul (121) ausgestaltet oder verbunden ist.

6. Pumpenantrieb (100) nach einem der vorangegangenen Ansprüche, wobei die Steuerelektronik (111) konfiguriert ist, mittels der Leuchteinrichtung (119) den laufenden Ladevorgang mit einer oder mehreren Farben, z. B. als Änderung der Farben von Rot über Gelb nach Grün, zu signalisieren bzw. den aktuellen Akkuladezustand anzuzeigen, und/oder bei oder nach Erreichen des Zustandes der vollständigen Ladung der Spannungsquelle oder des Akkus (105) auszuschalten.

7. Pumpenantrieb (100) nach einem der vorangegangenen Ansprüche, wobei die Steuerelektronik (111) konfiguriert ist, auf das Erkennen einer Bewegung des Pumpenantriebs (100) mittels des Bewegungssensors (123) hin von einem ersten Zustand der Steuerelektronik (111) in einen zweiten Zustand überzugehen, wobei die Steuerelektronik (111) konfiguriert ist, insbesondere auf das Erkennen einer Bewegung des Pumpentriebs (100) mittels des Bewegungssensors (123) hin, den Magnetabschnitt (107) des Pumpenantriebs (100) zu aktivieren, z. B. in den zweiten Zustand zu überführen und/oder den Ladezustand, insbesondere mittels der Leuchteinrichtung (119), anzuzeigen.

8. Pumpenantrieb (100) nach einem der vorangegangenen Ansprüche, wobei die Steuerelektronik (111) konfiguriert ist, insbesondere auf das Erkennen eines Pumpenabschnitts (300) mit dem Pumpenrotor (301) oder auf das Erkennen eines Pumpenrotors (301), z. B. mittels eines Hallsensors (125), hin, den Magnetabschnitt (107) des Pumpenantriebs (100) zu rotieren, insbesondere mit einer vorbestimmten Drehzahl, insbesondere unter Anzeigen der stattfindenden Rotation mittels der Leuchteinrichtung (119), z. B. in blau, z. B. mittels umlaufender Lichtemission.

9. Pumpenantrieb (100) nach einem der vorangegangenen Ansprüche, wobei die Steuerelektronik (111) konfiguriert ist, um mittels eines, insbesondere Low-power, Bewegungssensors (123) eine Bewegung des Pumpenantriebs (100) zu erkennen und in einen aktiven Zustand überzugehen und/oder einen Hinweis auf eine erfolgte Bewegung des Pumpenantriebs (100) an ein externes Gerät (500) zu übermitteln.

10. Pumpenantrieb (100) nach einem der vorangegangenen Ansprüche, wobei die Steuerelektronik (111) konfiguriert ist, insbesondere auf das Erkennen eines versiegenden oder versiegten Flüssigkeitsstroms hin, welcher durch eine Pumpe (405), deren Teil der Pumpenantrieb (100) ist, fließt, die maximale oder die voreingestellte Drehzahl zu reduzieren, vorzugsweise auf eine Drehzahl, welche ein Gleichgewicht herstellt zwischen der mittels der Pumpe (405) gepumpten Flüssigkeit und der Flüssigkeit, welche in die Pumpe (405) mittels Schwerkraft oder anderen Gründen nachläuft.

11. Pumpe (405), aufweisend einen Pumpenantrieb (100) nach einem der vorangegangenen Ansprüche, und mit wenigstens einem Pumpenabschnitt (300), welcher einen Pumpenrotor (301) aufweist, wobei der Pumpenabschnitt (300) mit dem Pumpenrotor (301) Teil eines Schlauchsatzes (4030) und/oder eines Disposables ist.

12. Pumpe (405) nach Anspruch 11, wobei der Pumpenabschnitt (300) mit dem Pumpenrotor (301) eine Einrichtung zum lösbaren Befestigen des Pumpenabschnitts (300) an einem Gehäuseabschnitt oder anderem Abschnitt einer Blutbehandlungsvorrichtung (1000), insbesondere im Fußbereich der Blutbehandlungsvorrichtung (1000), aufweist.

13. Pumpe (405) nach Anspruch 12, wobei die Einrichtung eine Klemmeinrichtung oder eine Rast- oder Clipeinrichtung (303) ist oder aufweist.

14. Pumpe (405) nach einem der Ansprüche 12 bis 13, wobei die Pumpe (405) keine Einrichtung zum Verbinden des Pumpenantriebs (100) mit dem Pumpenabschnitt (300), welcher einen Pumpenrotor (301) trägt, aufweist, abgesehen von einer optionalen Magnetverbindung.

15. Set, mit einem Pumpenantrieb (100) nach einem der Ansprüche 1 bis 10 und/oder mit einer Pumpe (405) nach einem der Ansprüche 11 bis 14, ferner aufweisend eine Induktionsladestation (600), welche konfiguriert ist, um das Aufliegen des Pumpenantriebs (100) gemäß einem der Ansprüche 1 bis 10 auf der Induktionsladestation (600) zu erkennen und ausgestaltet und konfiguriert ist, den Akku (105) des Pumpenantriebs (100) mittels Induktion zu laden, und/oder ein externes Gerät (500), konfiguriert, über das Wireless-Modul (121) mit der Steuerelektronik (111) in Signalverbindung zu stehen, um Signale, die Zustände, Funktionen und/oder Einstellungen der Steuerelektronik (111) oder des Akkus (105) zu empfangen und anzuzeigen.

16. Schlauchsatz (4030), aufweisend wenigstens einen Pumpenabschnitt (300), welcher einen magnetisch antreibbaren Pumpenrotor (301) trägt, wobei der Pumpenabschnitt (300) vorgesehen ist, um funktionell mit einem Pumpenantrieb (100) nach einem der Ansprüche 1 bis 10 verbunden oder zusammengebracht zu werden, wobei der Pumpenabschnitt (300) keine Einrichtung zum Verbinden des Pumpenabschnitts (300) mit dem Pumpenantrieb (100) aufweist, abgesehen von einer optionalen Magnetverbindung und/oder der Schwerkraft.

17. Medizinische Behandlungsvorrichtung, insbesondere Blutbehandlungsvorrichtung (1000), aufweisend eine Pumpe gemäß einem der Ansprüche 11 bis 14, ein Set nach Anspruch 15 und/oder einen Schlauchsatz (4030) gemäß Anspruch 16 oder hiermit jeweils verbunden.

## Claims

1. A medical pump drive (100) comprising at least:
- at least one housing (101) and one pump motor (103) provided in the housing (101);
- at least one rechargeable voltage source for storing electrical energy, or a battery (105), for supplying power to the pump motor (103);
- at least one magnet section (107) for magnetically coupling and/or magnetically driving a magnetically driven pump rotor (301); and
- at least one control electronics (111) programmed to activate the magnet section (107) of the pump drive (100);
**characterized in that** the control electronics (111) is designed as or connected to a motion sensor (123) configured to detect a position or motion of the medical pump drive (100) in space.

2. The pump drive (100) according to claim 1, comprising an induction charging coil (113) for charging the voltage source or the battery (105).

3. The pump drive (100) according to anyone of the preceding claims, wherein the housing (101) comprises:
- an installation section (115) for installing the pump drive (100) on an installation surface; and
- a connecting section (117) for connecting the pump drive (100) to a pump section (300) comprising a pump rotor (301);
wherein the installation section (115) and the connecting section (117) lie on opposite ends of the housing (101) or are associated therewith.

4. The pump drive (100) according to anyone of the preceding claims, designed with a, in particular colored lighting device (119), in particular designed as a ring, in particular as a LED or LED color ring, and/or in particular designed in or on the pump housing (101).

5. The pump drive (100) according to anyone of the preceding claims, wherein the control electronics (111) is designed as or connected to a wireless module (121).

6. The pump drive (100) according to anyone of the preceding claims, wherein the control electronics (111) is configured to signal the ongoing charging process with one or more colors, e.g. as a change of color from red via yellow to green using the lighting device (119) or to display the current battery charge state, and/or to switch off when or after reaching the state of full charge of the voltage source or battery (105).

7. The pump drive (100) according to anyone of the preceding claims, wherein the control electronics (111) is configured to change from a first state of the control electronics (111) into a second state, upon detecting a movement of the pump drive (100) by means of the motion sensor (123), wherein the control electronics (111) is configured, in particular upon detecting a movement of the pump drive (100) by means of the motion sensor (123), to activate the magnetic section (107) of the pump drive (100), e.g. to transfer it to the second state and/or to display the charge state, in particular by means of the lighting device (119).

8. The pump drive (100) according to anyone of the preceding claims, wherein the control electronics (111) is configured, in particular upon detecting a pump section (300) with the pump rotor (301) or upon detecting a pump rotor (301), e.g. by means of a Hall sensor (125), to rotate the magnetic section (107) of the pump drive (100), in particular at a predetermined rotational speed, in particular by displaying the current rotation by means of the lighting device (119), e.g. in blue, e.g. by means of peripheral light emission.

9. The pump drive (100) according to anyone of the preceding claims, wherein the control electronics (111) is configured to detect a movement of the pump drive (100) by means of a motion sensor (123), in particular a low-power motion sensor, and to change to an active state and/or to transmit an notification about a movement of the pump drive (100) to an external device (500).

10. The pump drive (100) according to anyone of the preceding claims, wherein the control electronics (111) is configured, in particular upon detecting a drying up or dried up liquid flow flowing through a pump (405), of which the pump drive (100) is part, to reduce the maximum or the preset rotational speed, preferably to a rotational speed establishing a balance between the liquid pumped by means of the pump (405) and the liquid flowing back into the pump (405) due to gravity or other reasons.

11. A pump (405), comprising a pump drive (100) according to anyone of the preceding claims, and comprising at least one pump section (300) having a pump rotor (301), wherein the pump section (300) having the pump rotor (301) is part of a tube set (4030) and/or of a disposable.

12. The pump (405) according to claim 11, wherein the pump section (300) having the pump rotor (301) comprises a device for the releasable fixing of the pump section (300) to a housing section or to another section of a blood treatment apparatus (1000), in particular at the foot area of the blood treatment apparatus (1000).

13. The pump (405) according to claim 12, wherein the device is or comprises a clamping device or a latching device or a clip device (303).

14. The pump (405) according to anyone of claims 12 to 13, wherein the pump (405) comprises no device, other than an optional magnet connection, for connecting the pump drive (100) to the pump section (300) which carries a pump rotor (301).

15. A set comprising a pump drive (100) according to anyone of claims 1 to 10 and/or a pump (405) according to anyone of claims 11 to 14, further comprising an induction charging station (600) configured to detect that the pump drive (100) according to anyone of claims 1 to 10, is lying on the induction charging station (600) and designed and configured to charge the battery (105) of the pump drive (100) via induction, and/or an external device (500) configured to be in signal communication with the control electronics (111) through the wireless module (121), in order to receive and display signals, the states, functions and/or settings of the control electronics (111) or of the battery (105).

16. A tube set (4030), comprising at least one pump section (300) carrying a magnetically drivable pump rotor (301), wherein the pump section (300) is provided to be functionally connected to or brought together with a pump drive (100) according to anyone of claims 1 to 10, the pump section (300) comprising no device, other than an optional magnet connection and/or gravity, for connecting the pump section (300) with the pump drive (100).

17. Amedical treatment apparatus, in particular a blood treatment apparatus (1000), comprising one pump according to anyone of claims 11 to 14, a set according to claim 15 and/or a tube set (4030) according to claim 16 or respectively connected herewith.

## Revendications

1. Un système d'entraînement de pompe médical (100) comprenant au moins:
- au moins un boîtier (101) ainsi qu'un moteur de pompe (103) prévu dans le boîtier (101);
- au moins une source de tension rechargeable pour le stockage d'énergie électrique, ou une batterie (105) pour l'alimentation du moteur de pompe (103);
- au moins une partie magnétique (107) pour le couplage magnétique et/ou l'entraînement magnétique d'un rotor de pompe à entraînement magnétique (301); et
- au moins un système électronique de commande (111) programmé pour activer la partie magnétique (107) du système d'entraînement de pompe (100);
**caractérisé en ce que** le système électronique de commande (111) est relié à, ou conçu sous la forme d'un capteur de mouvement (123) configuré pour détecter une position ou un mouvement du système d'entraînement de pompe médical (100) dans l'espace.

2. Le système d'entraînement de pompe (100) selon la première revendication, comprenant une bobine de charge par induction (113) pour charger la source de tension ou la batterie (105).

3. Le système d'entraînement de pompe (100) selon l'une quelconque des revendications précédentes, où le boîtier (101) comprend:
- une section d'installation (115) pour installer le système d'entraînement de pompe (100) sur une surface d'installation; ainsi que
- une section de raccordement (117) pour relier le système d'entraînement de pompe (100) à une partie de pompe (300) ayant un rotor de pompe (301);
où la section d'installation (115) et la section de raccordement (117) sont situées à des extrémités opposées du boîtier (101) ou y sont associées.

4. Le système d'entraînement de pompe (100) selon l'une quelconque des revendications précédentes, comportant un dispositif lumineux (119), notamment de couleur, conçu notamment sous forme d'anneau, notamment sous forme de LED ou d'anneau LED de couleur, et/ou conçu notamment dans ou sur le boîtier de pompe (101).

5. Le système d'entraînement de pompe (100) selon l'une quelconque des revendications précédentes, où le système électronique de commande (111) est conçu comme un module sans fil (121) ou est relié à un tel.

6. Le système d'entraînement de pompe (100) selon l'une quelconque des revendications précédentes, où le système électronique de commande (111) est configuré pour signaler, au moyen du dispositif lumineux (119), le processus de charge en cours via une ou plusieurs couleur (s), par exemple par un changement de couleur du rouge au vert en passant par le jaune, ou pour afficher l'état de charge actuel de la batterie et/ou pour s'éteindre lorsque l'état de charge complète de la source de tension ou de la batterie (105) est atteint ou après qu'il ait été atteint.

7. Le système d'entraînement de pompe (100) selon l'une quelconque des revendications précédentes, où le système électronique de commande (111) est configuré pour passer d'un premier état du système électronique de commande (111) à un second état suite à la détection d'un mouvement du système d'entraînement de pompe (100) au moyen du capteur de mouvement (123), où le système électronique de commande (111) est configuré, notamment suite à la détection d'un mouvement du système d'entraînement de pompe (100) au moyen du capteur de mouvement (123), pour activer la partie magnétique (107) du système d'entraînement de pompe (100), par exemple pour la faire passer dans le second état et/ou pour afficher l'état de charge, notamment au moyen du dispositif lumineux (119).

8. Le système d'entraînement de pompe (100) selon l'une quelconque des revendications précédentes, où le système électronique de commande (111) est configuré, notamment suite à la détection, par exemple au moyen d'un capteur à effet Hall (125), d'une partie de pompe (300) ayant le rotor de pompe (301) ou d'un rotor de pompe (301), pour faire tourner la partie magnétique (107) du système d'entraînement de pompe (100), notamment à une vitesse de rotation prédéterminée, notamment en affichant la rotation en cours au moyen du dispositif lumineux (119), par exemple en bleu, par exemple au moyen d'une émission de lumière périphérique.

9. Le système d'entraînement de pompe (100) selon l'une quelconque des revendications précédentes, où le système électronique de commande (111) est configuré pour détecter un mouvement du système d'entraînement de pompe (100) au moyen d'un capteur de mouvement (123), notamment d'un capteur de mouvement de faible puissance, et pour passer à un état actif, et/ou transmettre une indication d'un mouvement effectué du système d'entraînement de pompe (100) à un dispositif externe (500).

10. Le système d'entraînement de pompe (100) selon l'une quelconque des revendications précédentes, où le système électronique de commande (111) est configuré, notamment suite à la détection d'un flux de liquide en train de s'assécher ou déjà asséché s'écoulant au travers d'une pompe (405) dont le système d'entraînement de pompe (100) fait partie, pour réduire la vitesse de rotation maximale ou préréglée, de préférence à une vitesse de rotation établissant un équilibre entre le liquide pompé au moyen de la pompe (405) et le liquide refluant dans la pompe (405) sous l'effet de la gravité ou d'autres raisons.

11. Une pompe (405), comprenant un système d'entraînement de pompe (100) selon l'une quelconque des revendications précédentes, et comprenant au moins une partie de pompe (300) ayant un rotor de pompe (301), où la partie de pompe (300) ayant le rotor de pompe (301) fait partie d'un ensemble de tuyaux (4030) et/ou d'un dispositif à usage unique.

12. La pompe (405) selon la revendication 11, où la partie de pompe (300) ayant le rotor de pompe (301) comprend un dispositif de fixation amovible de la partie de pompe (300) à une partie de boîtier ou à une autre partie d'un appareil de traitement du sang (1000), notamment au niveau du pied de l'appareil de traitement du sang (1000).

13. La pompe (405) selon la revendication 12, où le dispositif est, ou comprend, un dispositif de serrage ou un dispositif d'encliquetage ou un dispositif de clipsage (303).

14. La pompe (405) selon l'une quelconque des revendications 12 à 13, où la pompe (405) ne comprend aucun dispositif, à l'exception d'une liaison magnétique optionnelle, pour relier le système d'entraînement de pompe (100) à la partie de pompe (300) supportant un rotor de pompe (301).

15. Un kit avec un système d'entraînement de pompe (100) selon l'une quelconque des revendications 1 à 10 et/ou une pompe (405) selon l'une quelconque des revendications 11 à 14, comprenant en outre une station de charge par induction (600) configurée pour détecter l'appui du système d'entraînement de pompe (100) selon l'une quelconque des revendications 1 à 10, sur la station de charge par induction (600), et étant conçue et configurée pour charger la batterie (105) du système d'entraînement de pompe (100) par induction, et/ou un dispositif externe (500) étant configuré pour être en communication de signal avec le système électronique de commande (111) via le module sans fil (121), afin de recevoir et d'afficher des signaux, les états, des fonctions et/ou des réglages du système électronique de commande (111) ou de la batterie (105).

16. Un ensemble de tuyaux (4030), comprenant au moins une partie de pompe (300), supportant un rotor de pompe à entraînement magnétique (301), où la partie de pompe (300) est prévue pour être reliée ou couplée de manière fonctionnelle à un système d'entraînement de pompe (100) selon l'une quelconque des revendications 1 à 10, la partie de pompe (300) ne comprenant aucun dispositif, à l'exception d'une liaison magnétique optionnelle et/ou de la gravité, pour relier la partie de pompe (300) au système d'entraînement de pompe (100).

17. Un appareil de traitement médical, notamment un appareil de traitement du sang (1000), comprenant une pompe selon l'une quelconque des revendications 11 à 14, un kit selon la revendication 15 et/ou un ensemble de tuyaux (4030) selon la revendication 16 ou respectivement relié à celle-ci/ceux-ci.
